(19) Europäisches Patentamt
European Patent Office
Office européen des brevets

(11) **EP 2 218 472 B2**

(12) **NEUE EUROPÄISCHE PATENTSCHRIFT**

Nach dem Einspruchsverfahren

(45) Veröffentlichungstag und Bekanntmachung des Hinweises auf die Entscheidung über den Einspruch:
**16.03.2022 Patentblatt 2022/11**

(45) Hinweis auf die Patenterteilung:
**14.09.2011 Patentblatt 2011/37**

(21) Anmeldenummer: **09001890.4**

(22) Anmeldetag: **11.02.2009**

(51) Internationale Patentklassifikation (IPC):
**A61M 1/16** *(2006.01)*     **G01J 3/42** *(2006.01)*
**G01N 21/33** *(2006.01)*

(52) Gemeinsame Patentklassifikation (CPC):
**A61M 1/16; A61M 1/1609; G01J 3/02; G01J 3/027; G01J 3/0286; G01J 3/42; G01N 21/274; G01N 21/278;** A61M 2205/3313; A61M 2205/3317; A61M 2205/3324; A61M 2205/3368; A61M 2205/50

(54) **Vorrichtung zur extrakorporalen Blutbehandlung**

Device for treating blood outside the body

Dispositif destiné au traitement du sang extracorporel

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(43) Veröffentlichungstag der Anmeldung:
**18.08.2010 Patentblatt 2010/33**

(73) Patentinhaber: **B. Braun Avitum AG**
**34212 Melsungen (DE)**

(72) Erfinder: **Ahrens, Jörn**
**34225 Baunatal (DE)**

(74) Vertreter: **Winter, Brandl - Partnerschaft mbB**
**Alois-Steinecker-Straße 22**
**85354 Freising (DE)**

(56) Entgegenhaltungen:
WO-A1-99/23479          WO-A1-99/62574
DE-A1-102005 063 263

- **FRIDOLIN I ET AL: "ON-LINE MONITORING OF SOLUTES IN DIALYSATE USING ABSORPTION OF ULTRAVIOLET RADIATION: TECHNIQUE DESCRIPTION" INTERNATIONAL JOURNAL OF ARTIFICIAL ORGANS, MILAN, IT, Bd. 25, Nr. 8, 1. August 2002 (2002-08-01), Seiten 748-761, XP008101248 ISSN: 0391-3988**
- **Nephzol Dial Transplant (2006) 21: 2225-2231, Advance Access Publication, 12 April 2006**
- **Journal of Remal Care 2007, DOI: 10.111/j.1755-6686.2007.tb00037.x**

EP 2 218 472 B2

**Beschreibung**

Die Erfindung betrifft eine Vorrichtung zur extrakorporalen Blutbehandlung nach dem Oberbegriff des Patentanspruchs 1.

Bei Patienten mit reduzierter bzw. überhaupt keiner Nierenfunktion werden Abfallprodukte, einschließlich toxischer Substanzen, mittels einer Nierenersatzbehandlung beseitigt, wobei das Blut des Patienten über einer Blutzuführleitung vom Patienten der künstlichen Niere bzw. dem Dialysator zugeführt wird. In der künstlichen Niere bzw. dem Dialysator wird das Blut des Patienten über eine semipermeable Membran mit Dialysierflüssigkeit in Kontakt gebracht. Die Dialysierflüssigkeit enthält unterschiedliche Salze in einer solchen Konzentration, dass die Abfallprodukte, einschließlich der toxischen Substanzen, mittels Diffusion und Konvektion durch die Membran aus dem Blut des Patienten zur Dialysierflüssigkeit geführt werden. Das so von den Abfallprodukten bereinigte Blut wird über eine an dem Dialysator angeschlossenen Blutabführleitung wieder in den Blutkreislauf des Patienten zurückgeführt.

Um das Ergebnis einer Nierenersatzbehandlung quantifizieren zu können, ist es notwendig, die Effizienz der Nierenersatzbehandlung unmittelbar bzw. online zu steuern. Deshalb wurde das so genannte Kt/V-Modell entwickelt. Der Kt/V-Wert ist dabei ein Parameter zur Bestimmung der Effektivität einer Nierenersatzbehandlung, wobei die Clearance K für den Volumenstrom der gereinigten harnpflichtigen Substanzen, t für die Behandlungszeit und V für das Verteilungsvolumen des Patienten steht Dabei sind sowohl K als auch V jeweils auf das auf das jeweilige Abfallprodukt bezogen. In der Regel wird die Effizienz bei einer Nierenersatzbehandlung anhand des Harnstoffes als Abfallprodukt beschrieben, so dass K die Harnstoffclearance und V das Harnstoffverteilungsvolumen des Patienten, welches im Wesentlichen dem Körperwassers des Patienten entspricht, beschreibt

Aus der EP 1 083 948 A1 und der EP 2 005 982 A1 ist es bekannt, mit Hilfe einer im Ablauf angeordneten Messeinrichtung spektralphotometrisch unter Verwendung von UV-Strahlung und deren Absorption durch harnpflichtige Substanzen in der Dialysierflüssigkeit den Kt/V-Wert bzw. die Reduktionsrate RR für ein bestimmtes Abfallprodukt während der Nierenersatzbehandlung zu bestimmen.

Bei diesen bekannten Vorrichtungen hat sich allerdings herausgestellt, dass eine gleichmäßige Strahlungsintensität der Strahlungsquelle und eine gleichmäßige Empfindlichkeit des Detektorsystems weder über die Betriebszeit der Strahlungsquelle noch während einer einzelnen Nierenersatzbehandlung gewährleistet werden konnte. Somit basiert die Absorptionsmessung in der verbrauchten Dialysierflüssigkeit während verschiedener Behandlungen und auch während einer Behandlungszeit auf veränderlichen Strahlungsintensitäten der Strahlungsquelle und/oder einem veränderten Ausgangssignal bei konstantem Eingangssignal des Detektorsystems. Dies hat zur Folge, dass der auf der Absorptionsmessung basierende Kt/V-Wert bzw. die auf der Absorptionsmessung basierende Reduktionsrate RR für ein bestimmtes Abfallprodukt nicht den tatsächlichen Gegebenheiten entsprechen. Vielmehr ist die Absorptionsmessung in der verbrauchten Dialysierflüssigkeit und damit die Aussage hinsichtlich des Kt/V-Wertes bzw. der Reduktionsrate RR für ein bestimmtes Abfallprodukt verfälscht

Aufgabe der Erfindung ist es daher, eine Vorrichtung gemäß dem Oberbegriff des Patentanspruchs 1 derart weiterzubilden, dass durch die Absorptionsmessung eine zuverlässige und unverfälschte Aussage über den Kt/V-Wert bzw. der Reduktionsrate RR einer Nierenersatzbehandlung erhalten wird.

Eine weitere Aufgabe der Erfindung ist es, ein Verfahren zur Verfügung zu stellen, wodurch eine zuverlässige und unverfälschte Aussage über den Kt/V-Wert bzw. der Reduktionsrate RR einer Nierenersatzbehandlung erhalten wird.

Gelöst wird die vorrichtungsgemäße Aufgabe durch eine Vorrichtung mit den Merkmalen des Patentanspruchs 1. Vorteilhafte Ausgestaltungen der Endungen sind Gegenstand der Unteransprüche 2 bis 12.

Durch die Erfindung wird eine zuverlässige und unverfälschte Aussage über den Kt/V-Wert bzw. der Reduktionsrate RR einer Nierenersatzbehandlung erhalten, indem Mittel vorgesehen sind, um Alterung der Messeinrichtung wahrend der Betriebszeit und auftretende Anderungen der Intensitat der elektromagnetischen Strahlung der Strahlungsquelle und/oder der Empfindlichkeit des Detektorsystems wahrend Behandlungszeit zu kompensieren.

Es hat sich nämlich herausgestellt, dass die nachlassende Strahlungsintensität der Strahlungsquelle über deren Betriebszeit in erster Linie auf einen Alterungsprozess der Strahlungsquelle zurückzuführen ist. Da die Arbeitsintensität $I_0$ der Strahlungsquelle bei solchen Vorrichtungen in der Regel kleiner als die maximale Intensität $I_{max}$ der Strahlungsquelle ist, lässt sich das auf die Betriebszeit zurückzuführende Nachlassen der Strahlungsintensität einfach durch ein Nachführen der Strahlungsintensität der Strahlungsquelle kompensieren. Durch das Detektorsystem wird somit zu Beginn jeder Behandlung die Strahlungsintensität nach Absorption durch unverbrauchte Dialysierflüssigkeit gemessen. Sobald Abweichungen dieser Strahlungsintensität von der Strahlungsintensität des vordefinierten Sollwerts auftreten, wir diese Abweichung kompensiert. Diese Maßnahme führt dazu, dass die Absorptionsmessungen der erfindungsgemäßen Vorrichtung über deren gesamte Betriebszeit normierbar sind, da immer die gleiche Strahlungsintensität nach Absorption durch unverbrauchte Dialysierflüssigkeit zu Grunde gelegt wird.

Weiterhin hat sich auch gezeigt, dass während einer Nierenersatzbehandlung eine Konstanz eines Referenzsignal der Strahlungsintensität, welches durch Detektion der Strahlungsintensität ohne Absorption generiert wird, ebenfalls nicht gewährleistet werden kann. Wie sich herausgestellt hat, liegt die Ursache hierfür in Temperaturschwankungen sowohl an der Strahlungsquelle als auch am Detektorsystem. Erfindungsgemäß ist deshalb als Mittel zur Kompensation eine Temperaturregelung vorgesehen, durch welche die Temperatur der Strahlungsquelle auf einen vordefinierten Arbeitstemperaturbereich $\Delta T_1$ und/oder die Temperatur des Detektorsystems auf einen vordefinierte Arbeitstemperaturbereich $\Delta T_2$ regelbar ist Durch diese Maßnahme hat sich eine deutliche Stabilisierung sowohl der Signalintensität der Strahlungsquelle als auch der Empfindlichkeit des Detektorssystems gezeigt, wobei durch Kombination der beiden alternativen Maßnahme die Stabilität und damit die Aussagekraft der letztendlich erhalten Ergebnisse der Kt/V-Werte und der Reduktionsrate RR nochmals signifikant gesteigert werden kann.

Allein die Kompensation der Änderung der Intensität der elektromagnetischen Strahlung der Strahlungsquelle oder der Empfindlichkeit des Detektorsystems führt bereits zu deutlich verbesserten Aussagen über den Kt/V-Wert bzw. die Reduktionsrate RR für ein bestimmtes Abfallprodukt. Eine nochmals signifikante Verbesserung der dieser Aussagen lässt sich erreichen, wenn beide Maßnahmen - zum einen eine Kompensation der Änderung der Intensität der elektromagnetischen Strahlung der Strahlungsquelle und zum anderen eine Kompensation der Änderung Empfindlichkeit des Detektorsystems - in der erfindungsgemäßen Vorrichtung integriert sind.

Erfindungsgemäß ist vorgesehen, dass als Mittel zur Kompensation des Alterungsprozesses der Strahlungsquelle eine elektronische Regelung vorgesehen ist, mit deren Hilfe die Intensität I der elektromagnetischen Strahlung der Strahlungsquelle derart regelbar ist, dass am Detektorsystem vordefinierte Intensitäten $I_{44}$, nach Absorption durch unverbrauchte Dialysierflüssigkeit, und/oder $I_{45}$, ohne Absorption durch unverbrauchte Dialysierflüssigkeit, detektierbar sind.

Dabei hat es sich als vorteilhaft erwiesen, dass die elektronische Regelung als Regelkreis ausgebildet ist, da solche Regelkreise bereits technisch ausgereift und einfach handhabbar sind.

Da die Absorption von harnpflichtige Substanzen im W-Bereich und im Wesentlichen bei 280nm sehr gut ist, bietet es sich an, dass die Strahlungsquelle als eine Leuchtdiode ausgebildet ist, welche in ihrem Arbeitstemperaturbereich $\Delta T_1$ elektromagnetische Strahlung im Wesentlichen der Wellenlänge 280nm emittiert.

Vorteilhaft ist weiterhin, wenn das Detektorsystem aus wenigstens einem fotodetektor vorzugsweise aus zwei Fotodetektoren besteht. Bei Verwendung nur eines Fotodetektor muss allerdings davon ausgegangen werden, dass die Signalintensität der von der Strahlungsquelle emittierten Strahlung zeitlich nicht konstant ist, um die Absorption der Dialysierflüssigkeit immer auf derselben Basis zu bestimmen. Deutlich besser ist es deshalb zwei Detektoren zu verwenden, wobei einer die Intensität der Strahlungsquelle und einer die Intensität der Strahlung nach Durchgang durch die die verbrauchte Dialysierflüssigkeit misst

Ein besonders effektive bevorzugte Ausgestaltung der Erfindung besteht daher darin, dass im Strahlengang der elektromagnetischen Strahlung zwischen der Strahlungsquelle und dem Abfluss für verbrauchte Dialysierflüssigkeit ein teildurchlässiger Spiegel oder eine optische Einrichtung zur Strahlaufteilung oder -umlenkung angeordnet ist, so dass ein Teil der elektromagnetischen Strahlung durch die verbrauchte Dialysierflüssigkeit auf den ersten Fotodetektor und der restliche Teil direkt auf den zweiten Detektor geleitet wird.

Nach einer weiteren bevorzugten Ausgestaltung der Erfindung ist die Regelgröße des Regelkreises die Intensität der elektromagnetischen Strahlung am ersten Detektor und die Stellgröße der elektrische Strom der Strahlungsquelle, wobei die dann ermittelte Intensität am zweiten Detektor als Referenzwert für die jeweilige Nierenersatzbehandlung abspeicherbar ist Durch diese Maßnahme ist eine besonders gute Regelung der Strahlungsintensität der elektromagnetischen Strahlungsquelle während der gesamten Betriebszeit der Strahlungsquelle gewährleistet

In diesem Zusammenhang hat es sich als besonders vorteilhaft erwiesen, dass die am zweiten Detektor ermittelte Referenzgröße der Intensität während der jeweiligen Nierenersatzbehandlung die Regelgröße eines zweiten Regelkreises ist und dass der elektrische Strom der Strahlungsquelle die Stellgröße dieses zweiten Regelkreises ist Dadurch kann während einer Nierenersatzbehandlung eine Änderung dieser Referenzgröße im Wesentlichen ohne Zeitverzögerung kompensiert werden.

Um die Temperaturregelung möglichst einfach und effektiv zu gestalten, hat es sich als vorteilhaft erwiesen, dass die Temperaturregelung einen Kühlkörpers für die Leuchtdiode und/oder das Detektorsystem bzw. die Detektoren aufweist

Alternativ oder zusätzlich ist es natürlich auch möglich, dass die Temperaturregelung eine Wasserkühlung für die Leuchtdiode und/oder die Detektoren aufweist.

Weiterhin alternativ oder zusätzlich kann die Temperaturregelung einen oder mehrere Lüfter für die Leuchtdiode und/oder die Detektoren aufweisen.

Als besonders vorteilhaft hat es sich erwiesen, wenn die Temperaturregelung alternativ oder zusätzlich einen oder mehrere elektrothermische Wandler, beispielsweise Peltier-Elemente zur Temperaturregelung der Leuchtdiode und/oder der Detektoren aufweist

Weitere Ziele, Vorteile, Merkmale und Anwendungsmöglichkeiten der vorliegenden Erfindung ergeben sich aus der nachfolgenden Beschreibung der Ausführungsbeispiele anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen und deren Rückbeziehung.

**Es zeigen:**

Figur 1: eine schematische Darstellung eines Ausführungsbeispiels einer erfindungsgemäßen Vorrichtung,

Figur 2: eine schematische Darstellung eines Ausführungsbeispiels einer Messeinrichtung bzw. des Detektorsystems einer erfindungsgemäßen Vorrichtung,

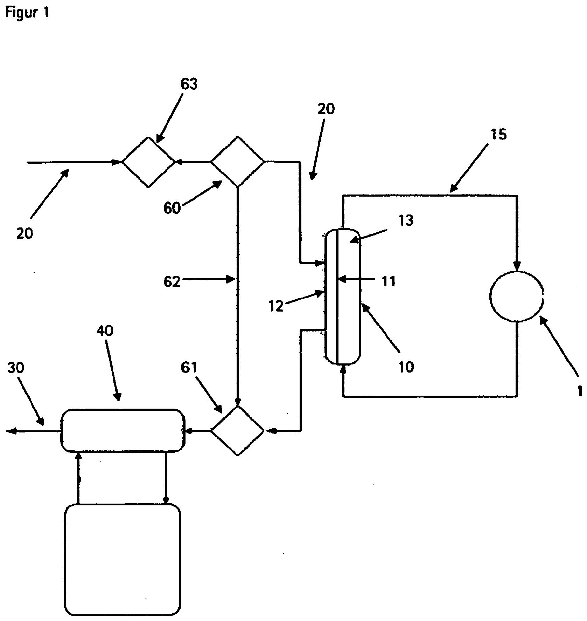

In Figur 1 ist ein Ausführungsbeispiel einer erfindungsgemäßen Vorrichtung in an einen Patienten 1 angeschlossenen Zustand dargestellt. Dabei ist der Patient 1 mittels einer Blutzuführleitung 14 mit einen Dialysator 10 verbunden. Von dem Dialysator führt eine Blutabführleitung 15 das gereinigte Blut wieder dem Blutkreislauf des Patienten zu.

Der Dialysator 10 ist mittels einer semipermeablen Membran 11 in zwei Kammern 12, 13 geteilt, wobei durch die erste Kammer 13 das zu reinigende Blut des Patienten 1 und durch die zweite Kammer 12 Dialysierflüssigkeit, welche in der Lage ist die im Blut des Patienten 1 enthaltenen Abfallprodukte und toxische Substanzen aufzunehmen, geführt wird. Der Transport der Abfallprodukte und toxischen Substanzen vom Blut des Patienten 1 in die Dialysierflüssigkeit erfolgt mittels Diffusion und Konvektion über die semipermeable Membran 11. Die Dialysierflüssigkeit wird mittels eines Zulaufs 20 der zweiten Kammer 12 des Dialysators 10 zugeführt. Dabei ist im Zulauf 20 eine Pumpe für die Förderung der Dialysierflüssigkeit ebenso vorgesehen wie ein Ventil 60, durch welches die Dialysierflüssigkeit anstatt in den Dialysator 10 über einen Bypass an diesem vorbei in einen Abfluss 30 für die Dialysierflüssigkeit geleitet werden kann. Im Abfluss 30 ist ebenfalls ein Ventil 61 angeordnet, welches mittels dem Bypass 62 mit dem Ventil 60 im Zulauf 20 verbunden ist

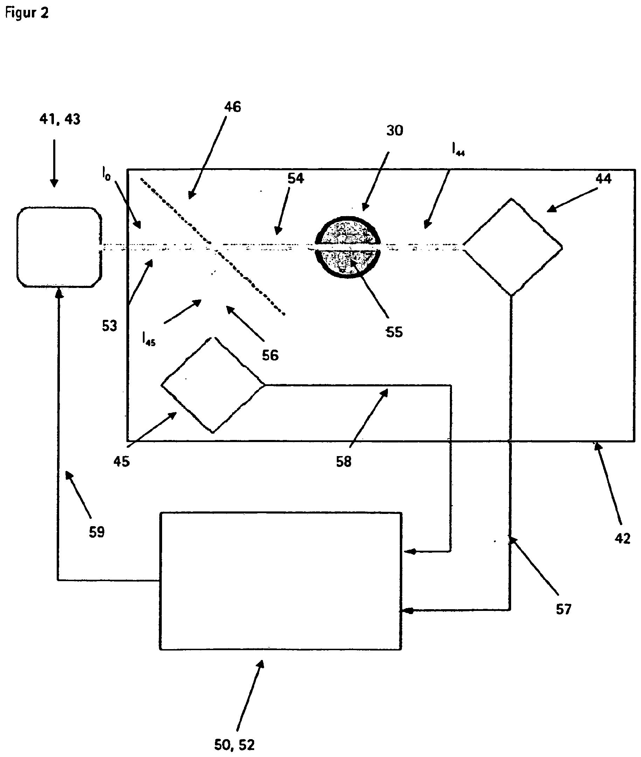

Nachdem im Dialysator 10 Abfallprodukte und toxische Substanzen vom Blut des Patienten 1 in die Dialysierflüssigkeit transportiert wurden, wird diese nunmehr verbrauchte Dialysierflüssigkeit über den Ablauf 30 entsorgt Im Ablauf 30 ist eine Messeinrichtung 40 angeordnet, mit welcher mittels einer Strahlungsquelle 41 für elektromagnetische Strahlung, insbesondere mit einer im UV-Bereich arbeitenden Leuchtdiode 43, und einem Detektorsystem 42, welches im vorliegenden Ausführungsbeispiel gemäß Figur 2 aus einem halbdurchlässigen Spiegel 46 und zwei Fotodetektoren 44, 45 besteht, die Absorption der verbrauchten Dialysierflüssigkeit bestimmt werden kann.

Die Funktionsweise der Messeinrichtung 40 und des Detektorsystems 42 ist folgende: Nach dem Prinzip der Zweistrahlspektroskopie wie sie in Figur 2 dargestellt ist emittiert die Leuchtdiode 43 als Strahlung 53 UV-Licht einer Wellenlänge von ca. 280nm, welches von dem halbdurchlässigen Spiegel 46 aufgeteilt wird. Ein Teil 54 der Strahlung 53 passiert den halbdurchlässigen Spiegel 46 und der restliche Teil 56 der Strahlung 53 wird von dem halbdurchlässigen Spiegel 46 auf den Detektor 45 reflektiert. Durch die in der verbrauchten Dialysierflüssigkeit 55 enthaltenen harnpflichtigen Substanzen wird ein gewisser Anteil der elektromagnetischen Strahlung des Teils 54 absorbiert. Der nicht von harnpflichtigen Subtanzen absorbierte Anteil des Teils 54 wird durch den Detektor 44 aufgezeichnet. Der am Detektor 45 aufgezeichnet Teil 56 der elektromagnetischen Strahlung ist somit unabhängig von harnpflichtigen Subtanzen in der Dialysierflüssigkeit 55 und über den halbdurchlässigen Spiegel direkt proportional zur Intensität I der Strahlungsquelle.

Da in der Dialysierflüssigkeit 55 im Abfluss 30 harnpflichtige Substanzen, die dem Blut im Dialysator entzogen wurden, enthalten sind und diese harnpflichtige Substanzen elektromagnetische Strahlung der Wellenlänge 280nm absorbieren kann mit Hilfe der am Detektor 44 bestimmten Intensität die Absorption von harnpflichtigen Substanzen im Abfluss 30 bestimmt werden. So ist der Verlauf der Absorption durch harnpflichtige Subtanzen während der Behandlung messbar, der als Grundlage zur Berechnung des Kt/V dient.

Mit steigender Konzentration von harnpflichtigen Subtanzen in der Dialysierflüssigkeit 55 wird das Signal am Detektor 44 verringert, da sich die Absorption erhöht. Aus dem Verlauf der Absorption wird dann eine e-Funktion ermittelt, aus welcher der Kt/V-Wert berechnet wird.

Um eine exakte Aussage über die Absorption während der Behandlungszeit zu bekommen, müssen allerdings Schwankungen der Intensität $I_o$ der Leuchtdiode 43 vermieden werden. Gewöhnlich erfolgt eine Kompensation von Schwankungen einer Lichtquelle bei einer Zweistrahlspektroskopie, wie Sie hier vorliegt, durch folgende Formel (bezogen auf die Absorption A), wobei die Intensitäten $I_{44}$ und $I_{45}$ an den Detektoren 44 und 45 in entsprechenden Signale umgewandelt werden:

$$A(t) = \log\left(\frac{U_{44}}{U_{45}} \cdot \frac{U_{45\_t}}{U_{44\_t}}\right)$$

mit $U_{44}$ = Signal am Detektor 44 mit unverbrauchter Dialysierflüssigkeit
$U_{45}$ = Signal am Detektor 45 zu Beginn
$U_{44\_t}$ = Signal am Detektor 44 zum Zeitpunkt t während der Therapie
$U_{45\_t}$ = Signal am Detektor 45 zum Zeitpunkt t während der Therapie

Mit der Vorrichtung gemäß den Figuren 1 und 2 wird die Intensität $I_0$ während der Therapie konstant gehalten, so dass $U_{45}=U_{45\_t} \sim I_0$, wobei $I_0$ im Arbeitsbereich der Leuchtdiode frei wählbar ist Damit reduziert sich die Gleichung für die Absorption A auf:

$$A(t) = \log\left(\frac{U_{44}}{U_{44\_t}}\right)$$

Die Absorption ergibt einen Kurvenverlauf, der durch eine e-Funktion beschrieben werden kann, aus der mit $A(t) = a*\exp(b*t)$ der $Kt/V = 0b*t$ berechnet werden kann.

Um die bei den bekannten Vorrichtungen auftretenden Messfehler, insbesondere durch Alterung und Temperaturinstabilität zu kompensieren gibt es zwei anspruchsgemäße Ansätze:

- Signalstabilität durch elektronische Regelung der emittierten elektromagnetischen Strahlung auf vordefinierte Level
- Temperaturstabilität durch Temperaturregelung

Die Problematik der Alterung kann insbesondere bei der Strahlungsquelle 41 bzw. der Leuchtdiode 43, dem Ablauf 30 sowie den Detektorsystem 42 bzw. den beiden Detektoren 44 und 45 auftreten und eine Veränderung der Eigenschaften bewirken.

Mit Hilfe der elektronischen Regelung ist es möglich, Veränderungen durch Alterung und Temperaturschwankungen sehr präzise zu kompensieren. Die elektromagnetische Strahlung der Leuchtdiode 43 verliert durch Alterung bei einem konstanten Strom während der Betriebszeit an Intensität und reagiert ebenfalls bei Erhöhung der Temperatur mit einer sinkenden elektromagnetischen Strahlung. Ebenso erfolgt eine Alterung an den Detektoren 44 Et 45. Der halbdurchlässige Spiegel beeinflusst durch Alterung neben der Durchlässigkeit auch das Verhältnis des Strahlengangs zwischen und $I_0$ und $I_{44}$ und $I_{45}$. Ebenso kann es beim Abfluss 30 zu einer konstanten Trübung kommen.

Durch einen vordefinierten Sollwert $I_{44\_Soll}$ der Intensität der elektromagnetische Strahlung am Detektor 44, bei dem die elektromagnetische Strahlung reine Dialysierflüssigkeit ohne harnpflichtige Stoffe im Abfluss 30 durchquert hat, wird der Messbereich beziehungsweise die Auflösung des Detektorsystems 42 bzw. des Detektors 44 optimal genutzt. Dadurch werden Veränderungen im System durch Alterung erkannt und kompensiert, wobei dadurch auch eine Überprüfung der Leistungsfähigkeit des Messsystems erfolgt Damit wird gewährleistet, das die Signalqualität, der Messbereich, die Messauflösung und die Reproduzierbarkeit während der gesamten Lebensdauer konstant ist

Die Gleichung für die Absorption ergibt sich daher mit dem Sollwert $I_{44\_Soll}$ zu:

$$A(t) = \log\left(\frac{U_{44\_Soll}}{U_{44\_t}}\right)$$

Die elektrische Regelung erfolgt in zwei Schritten:

Zu Beginn der Nierenersatzbehandlung vor Anschluss des Patienten bzw. im Bypass befindet sich reine Dialysierflüssigkeit ohne harnpflichtige Subtanzen im Abfluss 30. In diesem Betriebszustand erfolgt zunächst eine Regelung des elektrischen Stroms der Strahlungsquelle 41 als Stellgröße, so dass am Detektor 44 der vordefinierten Sollwert $I_{44\_Soll}$ der Strahlungsintensität als Eingangssignal detektiert. Bei Erreichen des vordefinierten Sollwerts $I_{44\_Soll}$ am Detektor 44 wird der Intensitätswert $I_{45}$ von Detektor 45 gespeichert und dient in einer zweiten Regelung während der folgenden Nierenersatzbehandlung als Sollwert $I_{45\_Soll}$. Es erfolgt also nun eine Regelung des elektrischen Stroms der Strahlungsquelle 41 bzw. der Leuchtdiode als Stellgröße auf den Intensitätswert $I_{45\_Soll}$ am **Detektor 45.**

Die Regelung erfolgt mit einem adaptiven Regler, der zunächst automatisiert die von der Leuchtdiode 43, den Detektor 44, dem teildurchlässigen Spiegel 46 sowie dem Abfluss 30 abhängige Übertragungsfunktion $F_{44}(43,44,46,30))=U_{44}$ und die von der Leuchtdiode 43, den Detektor 45 und dem teildurchlässigen Spiegel 46 abhängige Übertragungsfunktion $F_{45}(43,46,45))=U_{45}$ des Systems erfasst. Die Regelung kann ebenfalls mit jeder anderen Art von Regelung erfolgen, was jedoch einen langsameren Einschwingvorgang zur Folge hätte.

Der erste Regelvorgang zu Beginn der Therapie erfolgt vor dem Anschluss des Patienten 1 bzw. im Bypass 62, bei dem durch entsprechende Einstellung der Ventile 60 und 61 die reine Dialysierflüssigkeit an dem Dialysator 10 vorbeigeführt wird, mit reiner Dialysierflüssigkeit ohne harnpflichtige Subtanzen. Mit Hilfe der adaptiven Regelung und der Übertragungsfunktion $F_{44}$ erfolgt der Regelvorgang auf den vordefinierten Sollwert der Strahlungsintensität $1_{44\_Soll} \sim U_{44\_Soll}$ am Detektor 44. Damit erfolgt die Kompensation der Alterung durch Änderung der elektrischen Stromstärke der Leuchtdiode 43. Wenn nötig können ebenfalls die Verstärkungsfaktoren der elektronischen Detektorschaltungen an den Detektoren 44 und 45 angepasst werden, sofern die Signalqualität dies zulässt. Der dann anliegende Messwert an Detektor 45 wird als Sollwert $U_{45}$ für den zweiten Regelvorgang gespeichert und dient während der Therapie als Sollwert, um Temperaturschwankungen zu kompensieren. Dabei ist die Absorption A=0, da $U_{44} = U_{44t}$.

Nach dem Anschluss des Patienten 1 erfolgt die Messwertaufnahme von $U_{4t}$ am Detektor 44. Durch harnpflichtige Substanzen ändert sich der Messwert am Detektor 44 und die Absorption ergibt sich aus:

$$A(t) = \log\left(\frac{U_{44}}{U_{44\_t}}\right)$$

Neben der Kompensation von Temperaturschwankungen ermöglicht diese Vorgehensweise auch einen konstanten Messbereichs und ebenfalls eine gleich bleibende Signalqualität. Während einer Therapie, also während einer einzigen Nierenersatzbehandlung, ist eine Alterung der Detektoren 44 und 45 zu vernachlässigen. Das Systems wird während der Nierenersatzbehandlung dann auf den Sollwert $U_{45}$ am Detektor 45 geregelt, der unabhängig vom Dialysierflüssigkeitsfluss eine stabile und konstante emittierte elektromagnetische Strahlung ermöglicht. Damit kann der Temperaturdrift der Leuchtdiode 43 bzw. der Intensität $I_o$ kompensiert werden. Der Stellwert der Regelung ist der elektrische Strom der Leuchtdiode 43, der proportional zur Intensität $I_o$ der durch die Leuchtdiode 43 emittierten Strahlung ist. Eine reine Regelung auf den Detektor 44 durch einen Vorgabewert zu Beginn ist jedoch nicht sinnvoll, da Einflüsse während der Therapie nicht **kompensiert werden können.**

Die Vorgabe des vordefinierten Sollwert im ersten Regelungsprozess dient zur Definition des Messbereichs der Elektronik und definiert gleichzeitig die Messauflösung der Absorption des **Messsignals.**

In den Figuren nicht dargestellte Verstärkerschaltungen innerhalb derelektronischen Regelung 52 wandeln das Signal der Detektoren 44 und 45 in eine Messspannung um, für die Analog-Digital-Wandler mit einer Mikroprozessor-Messwertaufnahme zur Verfügung stehen. Diese Anpassung der Verstärkerschaltungen, welche nur vor jeder einzelnen Nierenersatzbehandlung erfolgen kann, könnte automatisch vor der Nierenersatzbehandlung parallel zu Regelung des Stroms der Leuchtdiode 43 erfolgen. Während der Behandlung ist allerdings nur eine Regelung des Stroms möglich.

Mit Hilfe der Temperaturregelung wird für die Leuchtdiode 43 und die Detektoren 44 und 45 eine optimale Betriebstemperatur erreicht. Bei hohen Temperaturen muss der Strom als Stellgröße der Leuchtdiode 43 erhöht werden, um die emittierte elektromagnetische Strahlung bei steigenden Temperaturen bei einer konstanten Intensität zu halten. Umgekehrt muss bei sinkenden Temperaturen der Strom als Stellgröße der Leuchtdiode 43 gesenkt werden. Eine Erhöhung des Stroms ist jedoch nur im Betriebsbereich der Leuchtdiode 43 möglich und beschleunigt den Alterungsprozess. In einem Dialysegerät treten erfahrungsgemäß enorme Temperaturschwankungen auf. Deshalb ist es sinnvoll Temperaturschwankungen durch eine Temperaturregelung 51 zu kompensieren, so dass die Vorrichtung in dem optimalen Temperaturbereich betrieben werden kann. Durch solche Maßnahmen wird auch der Alterungsprozess verlangsamt

Ziel der Temperaturregelung ist, die Leuchtdiode 43 und die Detektoren 44 & 45 im optimalen Temperaturbereich zu betreiben bzw. schnell den optimalen Temperaturbereich für diese Komponenten zu erreichen, damit während der Therapie eine Änderung der Temperatur auf ein Minimum reduziert werden kann. Nach der Desinfektion kann es durch Erhitzung des Systems zu erhöhten Temperaturen an der Messeinrichtung 40 bzw. der Leuchtdiode 43 und/oder dem Detektorsystem 42 bzw. den Detektoren 44 und 45 kommen. Ferner hat beim Einschalten der Vorrichtung aus dem kalten Zustand die Eigenerwärmung des Systems eine Abkühlung zur Folge, wodurch zu Beginn eine sehr geringe Temperatur vorhanden ist. Daher ist es notwendig die Temperatur so schnell wie möglich in den Betriebsbereich der Komponenten zu bringen, während die Vorrichtung zur Nierenersatzbehandlung vorbereitet wird, um zu Beginn der Therapie bzw. während der Identifikation des System durch die oben beschriebene elektronische Regelung 52 bereits im Sollbereich der Temperatur zu sein. Damit ist es möglich auch sehr geringe Abweichungen der Detektoren 44 und 45 bezüglich eines Signaldrifts durch Temperaturänderungen auf ein Minimum zu reduzieren.

Die Temperaturstabilisierung erfolgt mit einem Kühlkörper mit Wasserkühlung, der die Flusstemperatur der Dialysierflüssigkeit direkt mit dem Kühlkörper 43 der LED und/oder die Detektoren 44&45 koppelt. Die Wärmekapazität der Dialysierflüssigkeit ist deutlich höher als die des Kühlkörpers der Leuchtdiode 43 und definiert daher die Temperatur, was ohne zusätzlichen technischen Aufwand möglich ist. Damit ist es möglich die Temperatur näherungsweise konstant im Betriebsbereich der Komponenten zu halten und das System schnell in den optimalen Temperaturbereich zu bringen.

Ohne enormen zusätzlichen Aufwand ist es jedoch nicht möglich die Temperatur so stabil zu halten, dass sie keinen weiteren Einfluss auf die Intensität der durch die Leuchtdiode 43 emittierte Strahlung hat. Daher muss parallel noch eine Stabilisierung der Leuchtintensität $I_o$ mit der oben genannten elektronischen Regelung 52 erfolgen.

Eine Kühlung kann ebenfalls mit anderen aktiven und passiven Kühlmethoden durchgeführt werden. Als passive Kühlung kann die Leuchtdiode 43 bzw. die Detektoren 44 und 45 über das Gehäuse oder eine Wasserkühlung temperaturstabilisiert werden. Als aktive Kühlung ist der Einsatz eines Lüfters möglich, der die Temperatur abhängig von der Umgebungstemperatur regeln kann. Ebenso ist eine direkte Regelung mit einem Peltier-Element oder ähnlichen elektrothermischen Wandlern zur Temperaturstabilisierung möglich.

Bezugszeichenliste

1 --     Patient
10 --    Dialysator
11 --    Membran
12 --    Kammer
13 --    Kammer
14 --    Blutzuführleitung
15 --    Blutabführleitung
20 --    Zulauf
30 --    Ablauf
40 --    Messeinrichtung
41 --    Strahlungsquelle
42 --    Detektorsystem
43 --    Leuchtdiode
44 --    Detektor
45 --    Detektor
46 --    halbdurchlässiger Spiegel
50 --    Mittel
51     -- Temperaturregelung
52 --    elektronische Regelung
53 --    Strahlengang
54 --    Teil der Strahlung
55 --    verbrauchte Dialysierflüssigkeit
56 --    Teil der Strahlung
57 --    Signalleitung
58 --    Signalleitung
59 --    Signalleitung
60 --    Ventil
61 --    Ventil
62 --    Bypass
63 --    Pumpe

**Patentansprüche**

1. Vorrichtung zur extrakorporalen Blutbehandlung mit einem Dialysator (10), der durch eine semipermeable Membran (11) in eine erste und zweite Kammer geteilt ist, wobei die erste Kammer (12) in einem Dialysierflüssigkeitsweg angeordnet ist und die zweite Kammer (13) mittels einer Blutzuführleitung (14) und einer Blutabführleitung (15) mit dem Blutkreislauf eines Patienten (1) verbindbar ist,

    - einem Zulauf (20) für frische Dialysierflüssigkeit,
    - einem Ablauf (30) für verbrauchte Dialysierflüssigkeit,
    - einer in dem Ablauf (30) angeordneten Messeinrichtung (40) zur Bestimmung der Absorption der durch den Ablauf (30) fließenden verbrauchten Dialysierflüssigkeit, wobei die Messeinrichtung (40) wenigstens eine Strahlungsquelle (41) für im Wesentlichen monochromatische elektromagnetische Strahlung sowie ein Detektorsystem (42) zur Detektion der Intensität der elektromagnetischen Strahlung aufweist, **dadurch gekennzeichnet, dass**

Mittel (50) vorgesehen sind, um auftretende Änderungen der Intensität der elektromagnetischen Strahlung der Strahlungsquelle (41) und/oder der Empfindlichkeit des Detektorsystems (42) zu kompensieren, wobei
als Mittel (50) eine Temperaturregelung (51) vorgesehen ist, durch welche die Temperatur der Strahlungsquelle (41) auf vordefinierte Arbeitstemperaturen T1 und/oder die Temperatur des Detektorsystems (42) auf vordefinierte Arbeitstemperaturen T2 regelbar ist, wobei für das Mittel (50) zusätzlich eine elektronische Regelung (52) vorgesehen ist, mit deren Hilfe die Intensität I der elektromagnetischen Strahlung der Strahlungsquelle (41) derart regelbar ist, dass am Detektorsystem (42) vordefinierte Intensitäten $I_{44}$, nach Absorption durch unverbrauchte Dialysierflüssigkeit, und/oder $I_{45}$, ohne Absorption durch unverbrauchte Dialysierflüssigkeit, detektierbar sind.

2. Vorrichtung nach Anspruch 1, **dadurch gekennzeichnet, dass** die elektronische Regelung (52) als Regelkreis ausgebildet ist

3. Vorrichtung nach einem der vorherigen Ansprüche, **dadurch gekennzeichnet, dass** die Strahlungsquelle (41) als eine Leuchtdiode (43) ausgebildet ist.

4. Vorrichtung nach Anspruch 3, **dadurch gekennzeichnet, dass** die Leuchtdiode (43) im Wesentlichen elektromagnetische Strahlung der Wellenlänge 280nm emittiert.

5. Vorrichtung nach einem der vorherigen Ansprüche, **dadurch gekennzeichnet, dass** das Detektorsystem (42) aus wenigstens einem Fotodetektor vorzugsweise aus zwei Fotodetektoren (44, 45) besteht.

6. Vorrichtung nach Anspruch 5, **dadurch gekennzeichnet, dass** im Strahlengang der elektromagnetischen Strahlung zwischen Strahlungsquelle (41) und Abfluss (30) für verbrauchte Dialysierflüssigkeit ein teildurchlässiger Spiegel (46) oder eine optische Einrichtung zur Strahlaufteilung oder -umlenkung angeordnet ist.

7. Vorrichtung nach einem der Ansprüche 3 bis 6, **dadurch gekennzeichnet, dass** die Regelgröße die Intensität $I_{44}$ der elektromagnetischen Strahlung am ersten Detektor (44) ist und dass die Stellgröße der elektrische Strom der Strahlungsquelle (41, 43) ist und die Intensität $I_{45}$ am Detektor (45) als Referenzwert $I_{45}$ abspeicherbar ist.

8. Vorrichtung nach Anspruch 7, **dadurch gekennzeichnet, dass** die Referenzgröße $I_{45}$ die Regelgröße eines zweiten Regelkreises ist und dass die Stellgröße der elektrische Strom der Strahlungsquelle (41, 43) ist.

9. Vorrichtung nach einem der vorherigen Ansprüche, **dadurch gekennzeichnet, dass** die Temperaturregelung (51) einen Kühlkörpers für die Leuchtdiode (43) und/oder das Detektorsystem (42) bzw. die Detektoren (44, 45) aufweist.

10. Vorrichtung nach einem der Ansprüche 1 bis 7, **dadurch gekennzeichnet, dass** die Temperaturregelung (51) eine Wasserkühlung für die Leuchtdiode (43) und/oder die Detektoren (44, 45) aufweist.

11. Vorrichtung nach einem der Ansprüche 1 bis 8, **dadurch gekennzeichnet, dass** die Temperaturregelung (51) einen oder mehrere Lüfter für die Leuchtdiode (43) und/oder die Detektoren (44, 45) aufweist.

12. Vorrichtung nach einem der Ansprüche 1 bis 9, **dadurch gekennzeichnet, dass** die Temperaturregelung (51) einen oder mehrere elektrothermischen Wandler, beispielsweise Peltier-Elemente zur Temperaturregelung der Leuchtdiode (43) und/oder der Detektoren (44, 45) aufweist.

## Claims

1. Apparatus for the extracorporeal treatment of blood with a dialyzer (10), which is separated into a first and second chamber by a semipermeable membrane (11), wherein the first chamber (12) is disposed in a dialysis fluid pathway and the second chamber (13) is connectable to the blood circulation of a patient (1) by way of a blood inflow conduit (14) and a blood outflow conduit (15),

   - a feed (20) for fresh dialysis fluid,
   - a discharge (30) for spent dialysis fluid,
   - a measuring device (40) disposed in the discharge (30) for estimation of the absorption of the spent dialysis fluid flowing through the discharge (30), wherein the measuring device (40) has at least one radiation source (41) for substantially monochromatic electromagnetic radiation as well as a detector system (42) for detecting the intensity of the electromagnetic radiation, **characterized in that**

   means (50) are provided to compensate for changes that occur in the intensity of the electromagnetic radiation of the radiation source (41) and/or the sensitivity of the detector system (42), wherein the means (50) is a temperature control (51) for adjusting the temperature of the radiation source (41) to first pre-defined operating temperatures T1, and/or the temperature of the detector system (42) to second pre-defined operating temperatures T2, wherein for the means (50) an electronic control (52) is additionally provided with the aid of which the intensity I of the electromagnetic radiation of the radiation source (41) can be controlled such that pre-defined intensities $I_{44}$ after absorption by fresh dialysis fluid, and/or intensities $I_{45}$ without absorption by fresh dialysis fluid are detectable.

2. The apparatus according to claim 1, **characterized in that** the electronic control (52) is formed as a control circuit.

3. The apparatus according to any of the preceding claims, **characterized in that** the radiation source (41) is formed as a light emitting diode (43).

4. The apparatus according to claim 3, **characterized in that** the light emitting diode (43) substantially emits electromagnetic radiation of a wavelength of 280nm.

5. The apparatus according to any of the preceding claims, **characterized in that** the detector system (42) consists of at least one photo detector, preferably of two photo detectors (44, 45).

6. The apparatus according to claim 5, **characterized in that** in the optical path of the electromagnetic radiation between radiation source (41) and discharge (30) for spent dialysis fluid a partially transmissive mirror (46) or an optical device is arranged for beam division or beam deflection.

7. The apparatus according to any of claims 3 to 6, **characterized in that** the intensity $I_{44}$ of the electromagnetic radiation at the first detector (44) is the control variable and the electric current of the radiation source (41, 43) is the regulating variable and that the intensity $I_{45}$ at the detector (45) can be stored as reference value $I_{45}$.

8. The apparatus according to claim 7, **characterized in that** the reference value $I_{45}$ is the control variable of a second control circuit and that the electric current of the radiation source (41, 43) is the regulating variable of the second control circuit.

9. The apparatus according to any of the preceding claims, **characterized in that** the temperature control (51) has a cooling body for the light emitting diode (43) and/or the detector system (42) and/or the detectors (44, 45).

10. The apparatus according to any of claims 1 to 7, **characterized in that** the temperature control (51) has a water-cooling device for the light emitting diode (43) and/or the detectors (44, 45).

**11.** The apparatus according to any of claims 1 to 8, **characterized in that** the temperature control (51) has one or more fans for the light emitting diode (43) and/or the detectors (44, 45).

**12.** The apparatus according to any of claims 1 to 9, **characterized in that** the temperature control (51) has one or more electrothermic converters, e.g. Peltier elements, for controlling the temperature of the light emitting diode (43) and/or the detectors (44, 45).

**Revendications**

**1.** Dispositif pour le traitement extracorporel de sang ayant un dialyseur (10) qui est séparé dans une première et une deuxième chambre par une membrane hémiperméable (11), dans lequel la première chambre (12) est disposée dans une voie pour le fluide de dialyse et la deuxième chambre (13) est connectable à la circulation sanguine d'un patient (1) par un conduit pour l'afflux sanguin (14) et un conduit par l'écoulement sanguin (15),

- une alimentation (20) pour le fluide de dialyse frais,
- un conduit (30) pour le fluide de dialyse usé,
- un équipement de mesurage (40) disposé dans le conduit (30) pour déterminer l'absorption du fluide de dialyse usé qui coule par le conduit (30), dans lequel l'équipement de mesurage (40) a au moins une source de rayonnement (41) pour un rayonnement électromagnétique sensiblement monochromatique ainsi qu'un système détecteur (42) pour détecter l'intensité du rayonnement électromagnétique, **caractérisé en ce que**

des moyens (50) sont pourvus pour compenser des changements qui se produisent dans l'intensité du rayonnement électromagnétique de la source de rayonnement (41) et/ou la sensibilité du système détecteur (42), dans lequel comme moyen (50) un régulateur de température (51) est pourvu par lequel la température de la source de rayonnement (41) peut être ajustée à une température de fonctionnement T1 prédéfinie et/ou la température du système détecteur (42) à une température de fonctionnement T2 prédéfinie, dans lequel pour moyen (50) une commande électronique (52) est en outre pourvue par laquelle l'intensité I du rayonnement électromagnétique de la source de rayonnement (41) est ajustable de telle manière que dans le système détecteur (42) sont détectables des intensités prédéfinies $I_{44}$, après l'absorption par le fluide de dialyse non usé, et/ou $I_{45}$, sans absorption par le fluide de dialyse non usé.

**2.** Dispositif selon la revendication 1, **caractérisé en ce que** la commande électronique (52) est réalisée comme un circuit de commande.

**3.** Dispositif selon une des revendications précédentes, **caractérisé en ce que** la source de rayonnement (41) est réalisée en forme d'une DEL (43).

**4.** Dispositif selon la revendication 3, **caractérisé en ce que** la DEL (43) émet sensiblement du rayonnement électromagnétique d'une longueur d'onde de 280 nm.

**5.** Dispositif selon une des revendications précédentes, **caractérisé en ce que** le système détecteur (42) se compose d'au moins un photodétecteur et préférentiellement de deux photodétecteurs (44, 45).

**6.** Dispositif selon la revendication 5, **caractérisé en ce qu'**un miroir semi-transparent (46) ou un équipement optique est monté dans le trajet optique du rayonnement électromagnétique entre la source de rayonnement (41) et le conduit (30) pour le fluide de dialyse usé pour la séparation ou la déflection de faisceau.

**7.** Dispositif selon une des revendications 3 à 6, **caractérisé en ce que** l'intensité $I_{44}$ du rayonnement électromagnétique au premier détecteur (44) est la grandeur commandée et le courant électrique de la source de rayonnement (41, 43) est la grandeur de commande et l'intensité $I_{45}$ au détecteur (45) est sauvegardable comme valeur de référence $I_{45}$.

**8.** Dispositif selon la revendication 7, caractérisé en ce la valeur de référence $I_{45}$ est la grandeur commandée d'un deuxième circuit de commande et le courant électrique de la source de rayonnement (41, 43) est la grandeur de commande.

**9.** Dispositif selon une des revendications précédentes, **caractérisé en ce que** le régulateur de température (51) a un refroidisseur pour la DEL (43) et/ou le système détecteur (42), respectivement les détecteurs (44, 45).

**10.** Dispositif selon une des revendications 1 à 7, **caractérisé en ce que** le régulateur de température (51) a un refroidissement par eau pour la DEL (43) et/ou les détecteurs (44, 45).

**11.** Dispositif selon une des revendications 1 à 8, **caractérisé en ce que** le régulateur de température (51) a un ou plusieurs ventilateurs pour la DEL (43) et/ou les détecteurs (44, 45).

**12.** Dispositif selon une des revendications 1 à 9, **caractérisé en ce que** le régulateur de température (51) a un ou plusieurs convertisseurs thermoélectriques, pour exemple des modules Peltier, pour contrôler la température de la DEL (43) et/ou des détecteurs (44, 45).

Figur 1

Figur 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1083948 A1
- EP 2005982 A1